# EUROPEAN PATENT SPECIFICATION

(11) **EP 0 303 997 B1**
(45) Date of publication and mention of the grant of the patent: **19.10.1994**
(21) Application number: 88113186.6
(22) Date of filing: 13.08.1988
(51) Int. Cl.: C12N 15/00, C12N 9/50, C12P 21/02

(54) **Acyl-peptide hydrolase and methods of production and use**
Acyl-peptidhydrolase, Verfahren zu deren Herstellung und Verwendung
Hydrolase de peptides acylés, méthodes de fabrication et utilisation

(30) Priority: 21.08.1987 US 87936
(43) Date of publication of application: 22.02.1989
(73) Proprietor: THE GENERAL HOSPITAL CORPORATION, Boston, MA 02114 (US)
(72) Inventor: Smith, John A., Dr., Brookline, MA 02146 (US)
(74) Representative: Fischer, Hans-Jürgen, Dr.

(56) References cited:
- JOURNAL OF BIOCHEMISTRY, vol. 77, no. 1, 1975, pages 89-102; S. TSUNASAWA etal.: "Purification and properties of acylamino acid-releasing enzyme from rat liver"
- METHODS IN ENZYMOLOGY, vol. 106, 1984, pages 165-170, New York, US; S.TSUNASAWA et al.: "Amino-terminal acetylation of proteins: An overview"
- THE FASEB JOURNAL, vol. 2, no. 5, 20th March 1988, page A999, Lancaster, PA, US; L.-W. LIN et al.: "Structural organization of the rat acyl-peptide hydrolase gene"
- The Journal of Biological Chemistry, vol.264, no.15 (1989) pp.8892-8899 (= D2)

## Description

### BACKGROUND OF THE INVENTION

### Field of the Invention

The present invention is directed to the production of Acyl-Peptide Hydrolase by recombinant DNA technology. It is also directed to the use of the enzyme to catalyze hydrolysis of an acylated peptide or protein, and the reaction between a derivatized N^{α}-acetyl amino acid donor and an acceptor protein with a free α-NH₂ group.

### Brief Description of the Background Art

Since the discovery of an acetyl group at the amino-terminus of tobacco mosaic virus coat protein a number of N^{α}-acetylated proteins have been found in animals, plants, and their viruses, and also in bacteria and fungi. N^{α}-acetylation is therefore considered one of the typical modifications of proteins in living organisms. Moreover, in some eukaryotic cells, it has been suggested that more than 80% of the intracellular soluble proteins are N^{α}-acetylated (Brown, J.L., J. Biol. Chem. 254:1447-1449 (1979)).

The biological significance of N^{α}-acetylation of proteins is still an open question (see Tsunasawa et al., Method Enzymol. 106:165-170 (1984)). It has been proposed that this post-translational modification protects intracellular proteins from proteolysis. However, this does not hold true for all proteins. In the case of actin from slime mold, proteolytic degradation becomes slower when the protein is N^{α}-acetylated. In contrast, cat hemoglobin is degraded at the same rate irrespective of N^{α}-acetylation (Tsunasawa et al., 1984).

Recent results from DNA sequencing have shown that in structural genes for the secretory proteins that are N^{α}-acetylated, the carbon for the acetylated aminoterminal residue is directly preceded by the initiation codon without the insertion of additional codons for amino acids (Tsunasawa et al., 1984). Little effort has been made to understand the relationship between N^{α}-acetylation and the transport of secretory proteins across biological membranes. To understand completely the function of N^{α}-acetylation, it will be important to identify the N^{α}-acetylated amino acids in proteins and peptides on a microanalytical scale. For this purpose, removal of the N^{α}-acetyl group or the N^{α}-acetyl amino acid must be efficiently achieved.

Acyl-Peptide Hydrolase (APH) has been successfully used for the hydrolysis of N^{α}-acylated peptides. One such enzyme, which was purified from animal liver, can liberate the N^{α}-acetyl amino acid from rather short peptides derived from N^{α}-acetylated proteins (Tsunasawa et al., 1984). The substrate specificity is broad for the amino terminal residue. APH cleaves the N^{α}-terminal acetylated or formylated amino acid from a blocked peptide (Jones et al., BBRC 126:933 (1985)). This enzyme catalyzes the hydrolysis of a diverse number of peptides and displays different pH optima for certain substrates in doing so. This enzyme may also play a pivotal role in the processing of polypeptide chains during biosynthesis. The presence of the acetylated group has gone undetected on many proteins because in most cases the blocking group of the nascent polypeptide chain together with the first amino acid are removed sometime during biosynthesis. APH has been purified from rat liver (Tsunasawa et al., J. Biochem. 77:89-102 (1975)); from bovine liver (Gade et al., Biochim. Biophys. Acta 662:86-93 (1981)); from porcine liver (Tsunasawa et al., J. Biochem. 93:1217-1220 (1983)); from rat brain (Marks et al., J. Neurochem. 41:201-208 (1983)); and from human erythrocytes (Jones et al., Biochem. and Biophys. Res. Comm. 126:933-940 (1985)).

### SUMMARY OF THE INVENTION

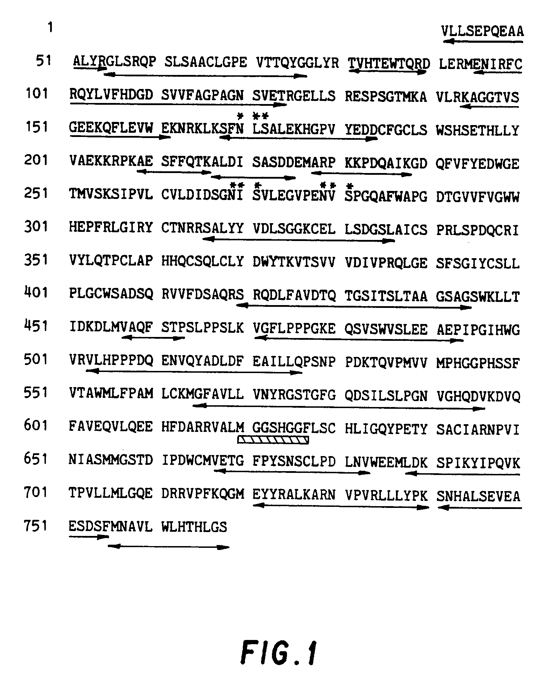

This invention is directed to a protein Acyl-Peptide Hydrolase (APH), which comprises the amino acid sequence of Figure 1. It is also directed to the production of APH by recombinant DNA technology, and to the utilization of APH in the hydrolysis or aminoacylation of peptides or proteins.

A recombinant DNA molecule coding for APH of the present invention may be used to transform any of a number of hosts, creating new sources and unlimited supplies of APH. It is a purpose of this invention to provide new sources of substantially pure APH which would be available in unlimited supply.

Additionally, this invention encompasses the use of the enzyme to catalyze the hydrolysis of an N^{α}-acylated protein, or the reaction between an N^{α}-acetyl amino acid donor and an acceptor protein with a free α-NH₂ group.

Therefore, additional purposes of this invention are to provide a means of hydrolysis of N-acylated proteins, and of amino-acylating any polypeptide or protein from an N^{α}-acetyl amino acid donor and an acceptor with a free α-NH₂ group, by the use of APH.

### DESCRIPTION OF THE FIGURES

Figure 1 illustrates the amino acid sequence of APH. The singly underscored portions and the cross-hatched portion are those areas sequenced both by Edman degradation of APH typsin and cyanogen bromide fragments, and by dideoxy-chain termination sequencing of clone APH36.1 cDNA. The asterisks indicate potential sites of glycosylation. The hatched portion represents the active site, which contains both a serine and a histidine.

Figure 2 indicates the APH peptide from which the sequences for degenerate probes YS17.2 and YS20.1 originated.

Figure 3 illustrates the scheme for screening a cDNA library with probes, in order to identify and isolate the gene encoded for APH.

Figure 4 illustrate the DNA sequence for the phage DNA insert of the APH36.1 clone, excluding the rat serum albumin sequence, plus additional 3′ untranslated sequence from rat liver clone APH36.1.

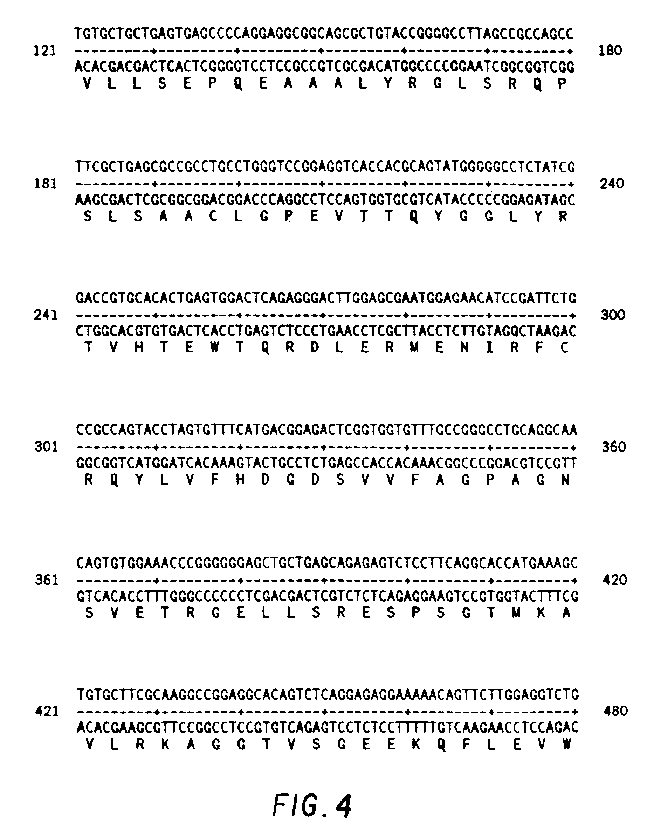

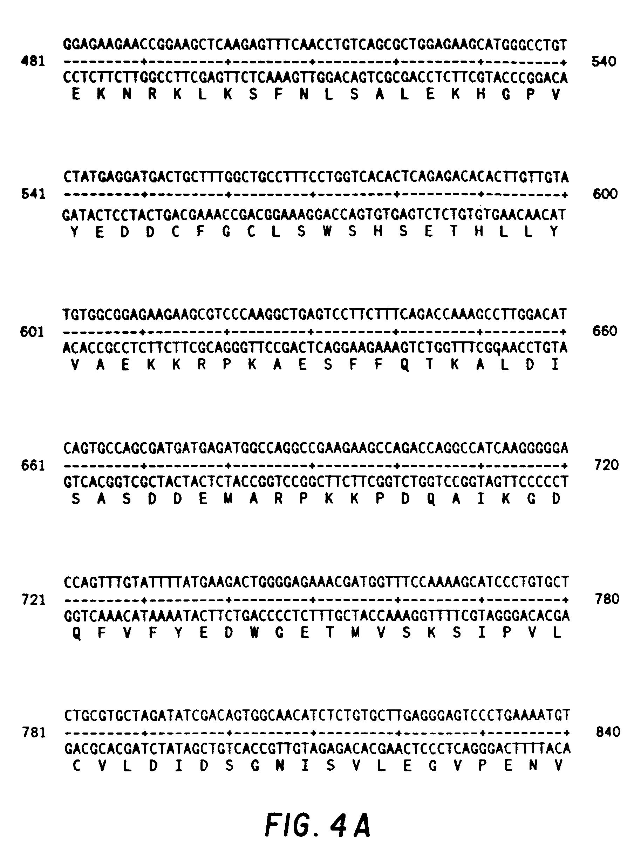

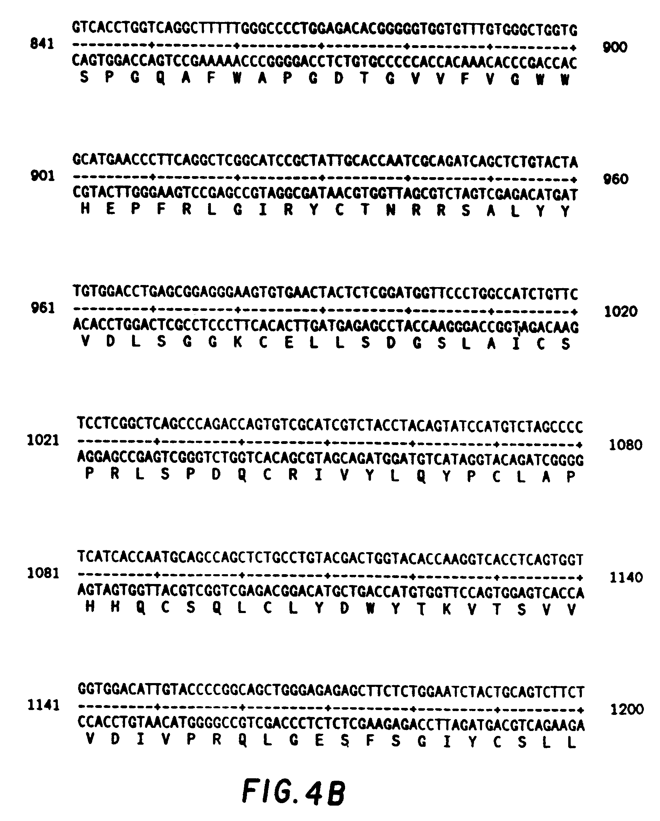

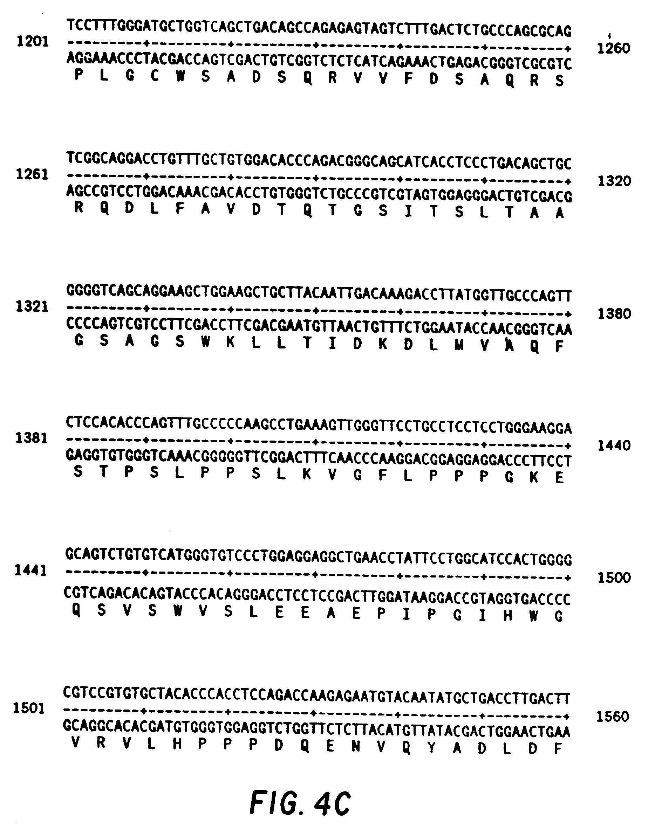

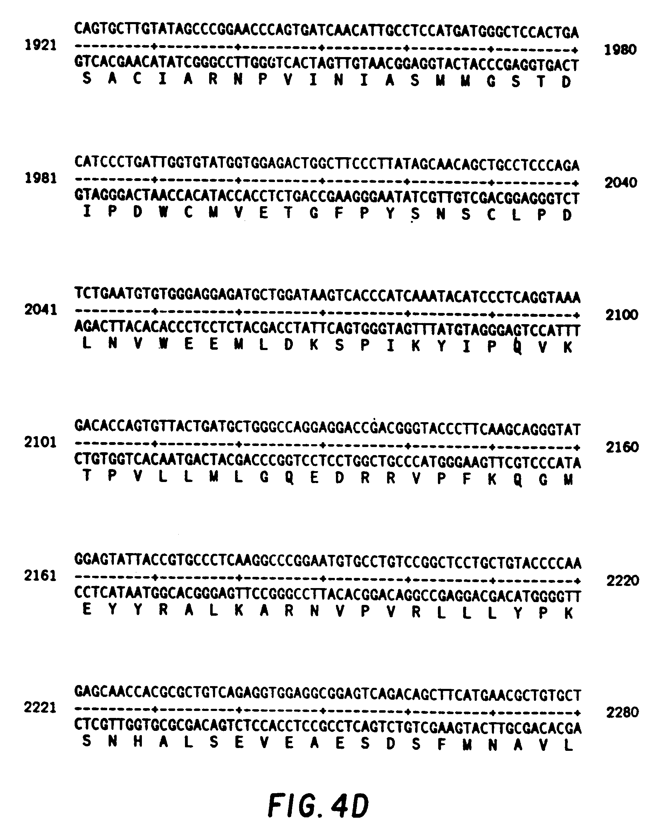

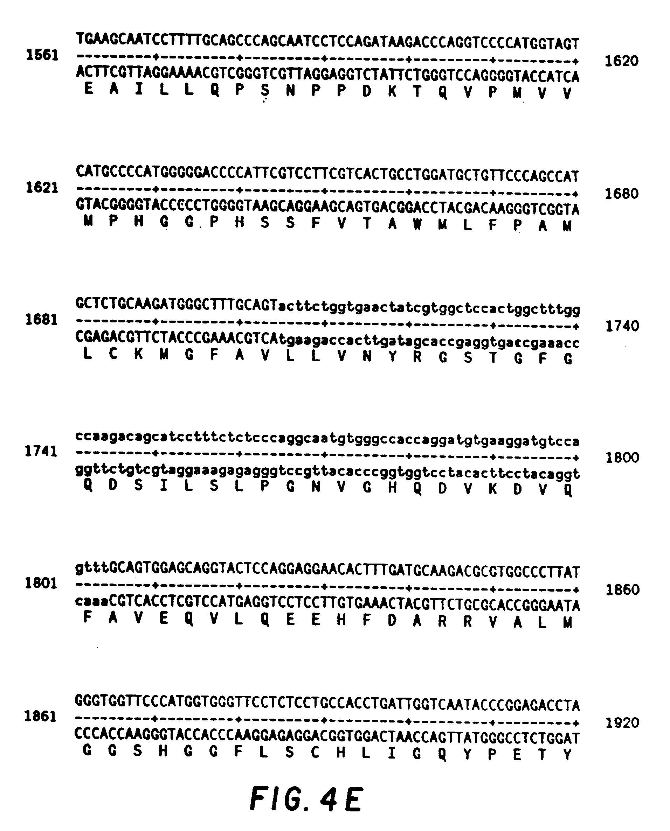

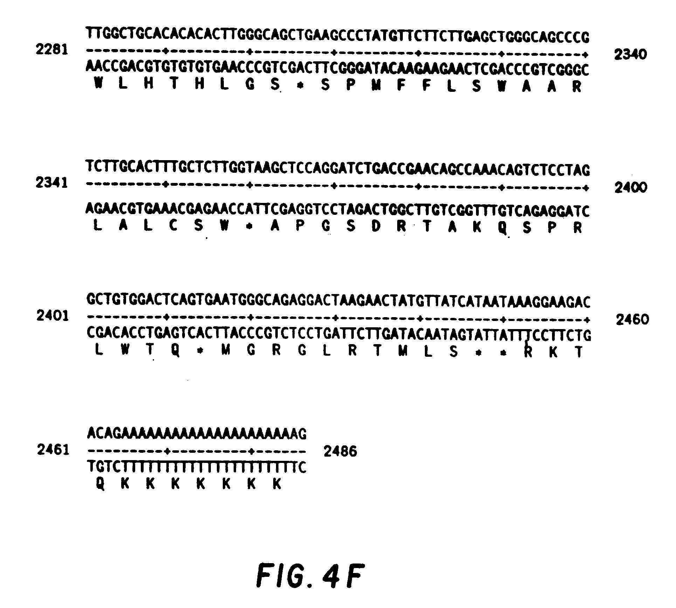

In Figures 1 and 4, the amino acids have been designated by single letters of the alphabet such that: A = Alanine, B = Aspartic Acid or Asparagine, C = Cysteine, D = Aspartic Acid, E = Glutamic Acid, F = Phenylalanine, G = Glycine, H = Histidine, I = Isoleucine, K = Lysine, L = Leucine, M = Methionine, N = Asparagine, P = Proline, Q = Glutamine, R = Arginine, S = Serine, T = Threonine, V = Valine, W = Tryptophan, Y = Tyrosine, Z = Glutamine or Glutamic Acid.

### DETAILED DISCUSSION OF THE INVENTION

### Definitions

To aid in the understanding of the specification and claims, including the scope to be given such terms, the following definitions are provided.

Transcription. The process of producing mRNA from a structural gene.

Translation. The process of producing a polypeptide from mRNA.

Expression. The process undergone by a structural gene to produce a polypeptide. It is a combination of transcription and translation.

Plasmid. A circular double-stranded DNA molecule that is not a part of the main chromosome of an organism containing genes that convey resistance to specific antibiotics. When the plasmid is placed within a unicellular organism, the characteristics of that organism may be changed or transformed as a result of the DNA of the plasmid. For example, a plasmid carrying the gene for tetracycline resistance (Tet^{R}) transforms a cell previously sensitive to tetracycline into one which is resistant to it. A cell transformed by a plasmid is called a "transformant."

Cloning Vehicle. A plasmid, phage DNA or other DNA sequences which are able to replicate in a host cell. The cloning vehicle is characterized by one or a small number of endonuclease recognition sites at which such DNA sequences may be cut in a determinable fashion without loss of an essential biological function of the DNA, which may contain a marker suitable for use in the identification of transformed cells. Markers, for example, are tetracycline resistance or ampicillin resistance. A cloning vehicle is often called a vector.

Recombinant DNA Molecules or Hybrid DNA. A molecule consisting of segments of DNA from different genomes which have been joined end-to-end outside of living cells and have a capacity to infect some host cell and be maintained therein.

Operator. A DNA sequence capable of interacting with the specific repressor, thereby controlling the transcription of adjacent gene(s).

Promoter. A DNA sequence in which RNA polymerase binds and initiates transcription of an adjacent gene(s).

Acyl-Peptide Hydrolase (APH). This term is meant to include an acyl-peptide hydrolase(s) from any species, which has the activity of releasing the N^{α}-terminal acylated amino acid from any protein or peptide in an in vivo or in vitro system. The term acyl-peptide hydrolase is also used in this invention to include any analogue, homologue, mutant or derivative of a naturally occurring acyl-peptide hydrolase, which cleaves the N^{α}-acetylated amino acid from the N^{α}-terminal portion of a peptide or a protein. The term is also meant to include fragments having less than the naturally-occurring number of amino acids, such a partial fragments of natural acyl-peptide hydrolases which retain the activity of cleaving the acylated amino acid from the N-terminal end of a protein or peptide. The term is also used to include any product which comprises the sequence of a naturally occurring acyl-peptide hydrolase or analogue thereof, together with one or more flanking amino acids, which show acyl-peptide hydrolase activity. The term acyl-peptide hydrolase also includes synonyms such as acyl-amino acid releasing factor, acyl-amino acid releasing enzyme, acyl-amino peptide hydrolase and acetylaminoacyl-p-nitroanilidase.

Substantially Pure Form. As used herein, the term "substantially pure" or "substantially purified" is meant to describe the protein which is substantially free of any compound normally associated with the factor in its natural state. The term is further meant to describe the factor which is homogeneous by one or more purity or homogeneity characteristics used by those of ordinary skill in the art. For example, a substantially pure factor will show constant and reproducible characteristics within a standard experimental deviations for parameters such as the following: molecular weight, chromatographic techniques and such other parameters. The term, however, is not meant to exclude artificial or synthetic mixtures of the factor with other compounds. The term is also not meant to exclude the presence of minor impurities which do not interfere with the biological activity of the factor, and which may be present, for example, due to incomplete purification.

### Products and Processes

The invention comprises an enzyme having the amino acid sequence designated in Figure 1, the genetic sequences coding for the enzyme, vehicles containing the genetic sequence, hosts transformed therewith, enzyme production by transformed host expression, and utilization of the enzyme in hydrolysis or in amino-acylation of peptides or proteins.

The molecular weight of rat liver APH, as estimated by gel filtration, is 290,000-320,000. There appear to be four identical subunits, with one active serine per subunit. The N^{α}-terminus of the APH is acylated. APH appears to be a serine portease, with a charge relay system involving serine, histidine and carboxyl groups. The environment of the active site differs from other proteases of the trypsin family, due to the presence of histidine. Although APH displays broad specificity for substrates, it cleaves Ac-Ala-, Ac-Ser-, and Ac-Met-containing peptides (the most common N^{α}-terminal residues) more effectively than other acylated dipeptides. APH has very low or no activity toward Ac-Trp-, Ac-Asp-, Ac-Glu, Ac-Arg-, Ac-Phe, and Ac-Pro- containing peptides.

Acyl-Peptide Hydrolase (APH) should be distinguished from N^{α}-acetyltransferase, which catalyzes the reaction in which a protein accepts the acetyl group from an acetyl-CoA (Tsunasawa et al., Methods in Embryology 106:165-170 (1984)). Acyl-Peptide Hydrolase should also be distinguished from Aminoacylase (Szajani; Acta Biochim. et Biophys. Acad. Sci. Hung. 15:223-228 (1980)), also known as α-N-Acylamino acid hydrolase (Gade et al. Biochim. et Biophys. Acta 662:86-93 (1981)).

Although APH has been isolated and purified from several sources, there has been no sequencing to date of APH. The present invention discloses that sequence (Figure 1).

The DNA sequence coding for APH may be derived from a variety of sources. For example, mRNA encoded for APH may be isolated from the tissues of any species that produces APH, by using the Northern blot method (Alwine et al., Method Enzymol. 68:220-242 (1979)), and labeled oligonucleotide probes. The mRNA may then be converted to cDNA by techniques known to those skilled in the art. The probes may be synthesized based on the known amino acid sequence of APH peptides.

Alternately, degenerative DNA probes maybe used to screen a DNA library of a species that produces APH, thereby isolating a clone that contains the DNA sequence encoding APH. The DNA library is created by the fragmentation, using one or more restriction endonucleases of the genomic DNA, followed by incorporation into vectors, and use thereof to transform host cells, which are then plated and screened.

The DNA probe may be labeled with a detectable group. Such detectable group can be any material having a detectable physical or chemical property. Such materials have been well-developed in the field of immunoassays and in general most any label useful in such methods can be applied to the present invention. Particularly useful are enzymatically active groups, such as enzymes (see Clin. Chem. 22:1243 (1976)), enzyme substrates (see British Pat. Spec. 1,548,741), coenzymes (see U.S. Pat. Nos. 4,230,797 and 4,238,565) and enzyme inhibitors (see U.S. Pat. No. 4,134,792); fluorescers (see Clin. Chem. 25:353 (1979)); chromophores; luminescers such as chemiluminescers and bioluminescers (see Clin. Chem. 25:512 (1979)); specifically bindable ligands; proximal interacting pairs; and radioisotopes such as ³H, ³⁵S, ³²P, ¹²⁵I and ¹⁴C. Such labels and labeling pairs are detected on the basis of their own physical properties (e.g., fluorescers, chromophores and radioisotopes) or their reactive or binding properties (e.g., enzymes, substrates, coenzymes, and inhibitors). For example, a cofactor-labeled probe can be detected by adding the enzyme for which the label is a cofactor and a substrate for the enzyme. For example, one can use an enzyme which acts upon a substrate to generate a product with a measurable physical property. Examples of the latter include, but are not limited to beta-galactosidase, alkaline phosphatase and peroxidase.

A DNA sequence encoding APH may be recombined with vector DNA in accordance with conventional techniques, including blunt-ended or stagger-ended termini for ligation, restriction enzyme digestion to provide appropriate termini, filling in of cohesive ends as appropriate, alkaline phosphatase treatment to avoid undesirable joining, and ligation with appropriate ligases.

To express APH, transcriptional and translational signals recognized by an appropriate host element are necessary. Eukaryotic hosts may be mammalian cells capable of culture in vitro, particularly leukocytes, more particularly myeloma cells or other transformed or oncogenic lymphocytes, e.g., EBV-transformed cells. Alternatively, non-mammalian cells may be employed, such as bacteria, fungi, e.g., yeast, filamentous fungi, or the like.

Possible hosts for APH production are mammalian cells, grown in vitro in tissue culture or in in vivo in animals. Mammalian cells may provide post-translational modifications to APH molecules including correct folding or glycosylation of the correct sites. Mammalian cells which may be useful as hosts include cells of fibroblast origin such as BERO or CHO-K1, or cells of lymphoid origin, such as the hybridoma SP2/O-AG14 or the myeloma P3x63Sgh, and their derivatives. Usually the APH construct will be part of a vector having a replication system recognized by the host cell.

In a preferred embodiment, a prokaryotic cell is transformed by a plasmid carrying the APH encoded gene. Bacterial hosts of particular interest include E. coli K12 strain 294 (ATCC 31446), E. coli X1776 (ATCC 31537), E. coli W3110 (F⁻, lambda⁻, prototropic (ATCC 27325)), and other enterobacteriacaes such as Salmonella typhimurium or Serratia marcescens, and various Pseudomona species. Under such conditions, the APH will not be glycosylated. The prokaryotic host must be compatible with the replicon and control sequences in the expression plasmid. Such a prokaryotic host with a plasmid containing the cDNA encoded for APH has been deposited on or before the filing date of this application at the American Type Culture Collection, Rockville, MD, USA.

In general, such vectors containing replicon and control sequences which are derived from species compatible with a host cell, are used in connection with the host. The vector ordinarily carries a replicon site, as well as specific genes which are capable of providing phenotypic selection in transformed cells. The expression of the APH encoded DNA can also be placed under control of other regulatory sequences which may be homologous to the organisms in its untransformed state. For example, lactose-dependent E. coli chromosomal DNA comprises a lactose or lac operon which mediates lactose utilization by elaborating the enzyme β-galactosidase. The lac control elements may be obtained from bacteriophage lambda plac5, which is infective for E. coli. The lac promoter-operator system can be induced by IPTG.

Other promoter/operator systems or portions thereof can be employed as well. For example, colicin El, galactose, alkaline phosphatase, tryptophan, xylose, tax, and the like can be used.

For a mammalian host, several possible vector systems are available for expression. One class of vectors utilize DNA elements which provide autonomously replicating extra-chromosomal plasmids, derived from animal viruses such as bovine papilloma virus, polyoma virus, adenovirus, or SB40 virus. A second class of vectors relies upon the integration of the desired gene sequences into the host chromosome. Cells which have stably integrated the introduced DNA into their chromosomes may be selected by also introducing one or markers which allow selection of host cells which contain the expression vector. The marker may provide for prototropy to an auxotrophic host, biocide resistance, e.g., antibiotics, or heavy metals, such as copper or the like. The selectable marker gene can either be directly linked to the DNA sequences to be expressed, or introduced into the same cell by co-transformation. Additional elements may also be needed for optimal synthesis of mRNA. These elements may include splice signals, as well as transcription promoters, enhancers, and termination signals. The cDNA expression vectors incorporating such elements include those described by Okayama, H., Mol. Cel. Biol. 3:280 (1983), and others.

A wide variety of transcriptional and translational regulatory sequences may be employed, depending on the nature of the host. The transcriptional and translational signals may be derived from viral sources, such as adenovirus, bovine papilloma virus, simian virus, or the like, where the regulatory signals are associated with a particular gene which has a high level of expression. Alternatively, promoters from mammalian expression products, such as actin, collagen, myosin, etc., may be employed. Transcriptional initiation signals may also be selected which allow for repression or activation, so that expression of the genes may be modulated. Of interest are regulatory signals which are temperature-sensitive so that varying the temperature, expression can be repressed or initiated, or are subject to chemical regulation, e.g., metabolite.

Once the vector or DNA sequence containing the constructs has been prepared for expression, the DNA constructs may be introduced to an appropriate host. Various techniques may be employed, such as protoplast fusion, calcium phosphate precipitation, electroporation or other conventional techniques. After the fusion, the cells are grown in media and screened for appropriate activities. Expression of the gene(s) results in production of the APH.

The host cells for APH production may be immortalized cells, primarily myeloma or lymphoma cells. These cells may be grown in an appropriate nutrient medium in culture flasks or injected into a synergistic host, e.g., mouse or rat, or immunodeficient host or host site, e.g., nude mouse or hamster pouch.

The APH of the invention may be isolated and purified in accordance with conventional conditions, such as extraction, precipitation, chromatography, affinity chromatography electrophoresis, or the like.

### Uses

APH, once produced and purified, can be used, for example, in a pharmaceutical manufacturing environment to hydrolyze an N^{α}-acylated peptide, or to amino-acylate the N^{α}-terminus of a peptide. The former is carried out in an aqueous solution, and makes refractory proteins susceptible to Edman sequencing. The latter may be performed in a near anhydrous environment, and is useful in reducing degradation of proteins to be used therapeutically. See the discussion following A. Klibinov, "Unconventional Catalytic Properties of Conventional Enzymes," in Basic Biology of New Developments in Biotechnology, pp. 497-518 (A. Hollander, ed. 1973), on the use of enzymes in biphaasic systems for organic synthesis.

The near anhydrous environment will alter the substrate specificity of APH, such that the aminoacylation of peptides takes place. Substrate specificity of an enzyme in organic solvents may be radically different from, and sometimes opposite to, those in water (see Zaks et al., J. Am. Chem. Soc. 108:2767-2768 (1986)). It has been shown that peptides can be synthesised by trypsin and α-chymotrypsin in solvents miscible or immiscible with water (see Pugniere et al., Proteins: Structure, Function, and Genetics 1:134-138 (1986)). Porcine pancreatic, yeast and mold lipases have been shown to vigorously act as catalysts in a number of nearly anhydrous solvents. The activity of the lipases in the organic media depends on the pH of the aqueous solution from which the lipase is recovered. The maximum lipase activity in the organic solvent coincides with the pH optimum of the enzymatic activity in water (see Zaks et al., Proc. Nat'l Acad. Sci. USA 82:3192-3196 (1985)). It has also been shown that a serine carboxypeptidase, such as carboxypeptidase Y derived from yeast, can synthesize a peptide from the reaction of an amino acid ester or amide or other substrate with an amino acid or other amine component (U.S. Patent No. 4,339,534).

Enzymes such as APH can vigorously function as catalysts in organic solvents, provided that some basic rules are followed. These rules include: (1) a proper choice of solvent (with hydrophobic ones being the best if they do not strip the essential layer of water from the enzyme molecule); (2) the use of an enzyme recovered from an aqueous solution of the pH optimal for enzymatic activity; and (3) elimination of diffusional limitations by vigorous agitation and fine dispersion of the enzyme powder in the organic solvent (see Zaks et al., 1986).

The reactants in the APH-catalyzed condensation reaction are acceptor polypeptides, e.g., proteins with a free N^{α}-terminal group, and a substrate such as a benzyl alcohol derivative of an acylated amino acid. Concentration of substrate needs to be sufficient to drive the amino-acylation reaction. The solvent chosen is a hydrophobic one that does not strip the essential layer of water molecules surrounding the enzyme. The APH, antecedent to its placement in the solvent, is recovered from an aqueous solution of the pH optimal for enzymatic activity. Dispersion of the fine APH powder in the solvent, and vigorous agitation is used to overcome diffusional limitations (Zaks et al., J. Am. Chem. Soc. 108:2767-2768 (1986)). Additionally, the organic environment will facilitate extraction of the APH due to enzyme insolubility in organic media (Zaks et al., Proc. Nat'l Acad. Sci. USA 82:3192-3196 (1985)).

APH may be suspended in its fine hydrated powder form, or may be immobilized on a carrier. The stability of enzymes toward inactivating agents, such as the monohydric alcohols is often enhanced by immobilization. It has been shown that trypsin and α-chymotrypsin, when immobilized on an insoluble alumina-phosphocolamine complex, demonstrate remarkable resistance toward organic solvents, including water-miscible monohydric alcohols (Pugniere et al., 1986). APH may be immobilized by methods known to those skilled in the art, on beads and other carriers, which then may be used in batches or columns.

Having now generally described this invention, the same will be better understood by reference to specific examples, which are included herein for purposes of illustration only, and are not intended to be limiting unless otherwise specified.

### EXAMPLES

### Example 1--Extraction and Purification of APH

Materials --DEAE Sepharose CL-6B, FPLC columns (Mono Q HR5/5, and Mono S HR5/5), Sephacryl S-300 superfine, Octyl-Sepharose, and Polybuffer 74 were from Pharmacia. Spherogel CAA-HIC column (0.46 x 10 cm) was from Beckman. Hydroxylapatite (Biogel HT) was from Bio-Rad. Glycerol was from BRL. Reactigel 6X was from Pierce. Amino acids (Ac-L-Ala) were from Sigma. All other chemicals were reagent grade or better.
Enzyme Purification --APH was purified from 300 g of rat liver (male, CD strain) as described by Tsunasawa et al., J. Biochem. (Tokyo) 77:89-102 (1975), except for the substitution of DEAE-Sepharose CL-6B and Sephacryl S-300 for DEAE cellulose and Sepharose 6B, respectively. The column sizes and gradients were also changed. For hydroxylapatite chromatography, the starting gradient was 5 mM phosphate buffer instead of 20 mM phosphate, and 10% glycerol was used in the gradient. Four mg of purified enzyme were obtained. During DEAE-Sepharose CL-6B chromatography, an increase in total activity was observed. In order to confirm the homogeneity of the protein from the Sephacryl S-300, additional chromatography was carried out: (i) ion-exchange chromatography with Pharmacia FPLC system on Mono Q and Mono S with various buffers at pH's between 5 and 8; (ii) hydrophobic interaction chromatography on Octyl-Sepharose and Spherogel CAA-HIC; (iii) chromatofocusing on Mono P with Polybuffer 74; and (iv) affinity chromatography using Ac-L-Ala--Sepharose, prepared from Reacti-Gel 6X (Pierce) and acetyl-L-alanine. In no case was further separation or increased activity observed.

**Table I**

| Purification of Acylpeptide Hydrolase from Rat Liver | | | | | |
|---|---|---|---|---|---|
| Step | Activity (unit) | Protein (mg) | Specific Activity (unit/mg) | Yield (%) | Purification |
| 1 Homogenate | 194 | 44200 | 0.00439 | 100 | 1.00 |
| 2 12000 x g Supernatant | 194 | 39400 | 0.00492 | 100 | 1.12 |
| 3 Ammonium Sulfate (20-50%) | 150 | 25400 | 0.00591 | 77 | 1.35 |
| 4 Heat Treatment | 139 | 2520 | 0.0552 | 65 | 11.5 |
| 5 DEAE-Sepharose | 208 | 29.3 | 7.10 | 108 | 1630 |
| 6 Hydroxylapatite | 148 | 5.90 | 25.1 | 76 | 5780 |
| 7 Sephacryl S-300 | 118 | 4.04 | 29.2 | 61 | 6090 |

### Example 2--Amino Acid Sequencing of Tryptic and Cyanogen Bromide Fragments of APH and Construction of Probes.

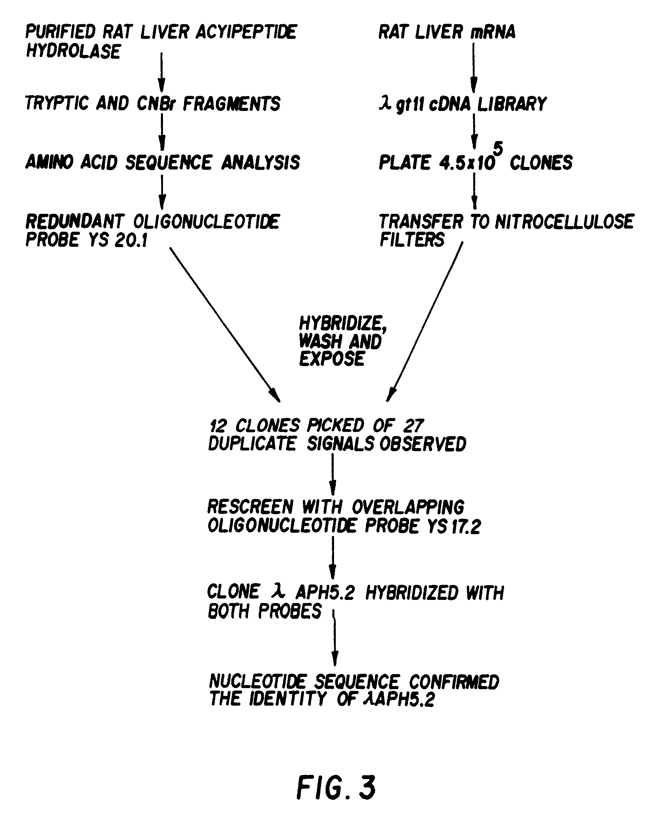

This procedure is illustrated in the left arm of Figure 3.
Materials --APH was purified as in Example 1. Purity was confirmed by SDS polyacrylamide gel electrophoresis by the method of Laemmli, Nature 227:680-685 (1970).

Cyanogen bromide, guanidine-HCl, 2-mercaptoethanol, trifluoroacetic acid (TFA), were obtained from Pierce. Acetonitrile (HPLC grade UV cut-off 188 nm) was from J.T. Baker. Trypsin treated with N-tosyl-PheCH₂Cl was purchased from Worthington. Bradform protein assay reagent and electrophoresis reagents were obtained from Bio-Rad, except for molecular weight markers and Tris, which were purchased from Sigma. Zwittergent 3-14 was from Calbiochem and [¹⁴-C] iodoacetic acid (9.8 mCi/mmol) was from New England Nuclear.

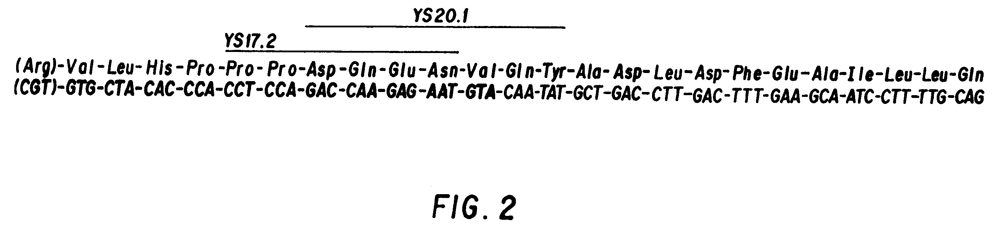

All other reagents were the purest grade that was commercially available.
Reduction of Disulfide Bonds and Alkylation with Iodoacetic Acid. The APH (3 nmol) was denatured in 0.5 M Tris-HCl (pH 8.5) containing 7 M guanidine HCl/2 mM EDTA, and reduced with 8-10 mM 2-mercaptoethanol under argon at a room temperature for 12 hr or at 37°C for 3 hr. To the mixture (0.19 ml), [¹⁴-C] iodoacetic acid (2.6 µmol in 30 µl 0.5 M Tris-HCl (pH 8.5)/7 M guanidine HCl/2 mM EDTA) was added and the reaction was carried out for 1 hr at 37°C in the dark. 2-Mercaptoethanol was then added to a final concentration of 0.2 M. The protein was desalted either by precipitating with four volumes of acetone/methanol (3:1 v/v) or by dialysis against 0.1 M acetic acid and lyophilized in a Savant concentrator/evaporator.
Cyanogen Bromide Cleavage. The carboxymethylated protein was dissolved in 70% formic acid (0.1-0.2 ml), to which 10-15 µl CNBr solution (100 mg/ml in 70% formic acid) was added. The mixture was incubated at room temperature for 24 hr and vacuum dried after the dilution with water. The CNBr-cleaved peptide fragments were purified by reversed-phase HPLC (RPLC) or further fragmented by tryptic digestion.
Digestion with Trypsin. The CNBr peptides were dissolved in 0.2 ml of 0.2 M ammonium bicarbonate containing 0.2% Zwittergent 3-14 and digested with trypsin (50 pmol) treated with N-tosyl-PheCH₂Cl for 20 hr at 37°C. The digest was vacuum dried and dissolved in 6 M guanidine HCl in 0.1% TFA for RPLC purification.
Purification of Peptide Fragments by Reversed-Phase HPLC. The peptides were purified by RPLC on a Beckman HPLC system 344, using a C₄ column (Vydac, 0.46 x 25 cm, 10 micron particle with 300 A^{o} pore) for CNBr fragments or a Phenyl column (Vydac, 0.46 x 25 cm, 5 micron particle with 300 A^{o} pore) for tryptic fragments. The crude peptide mixture was applied to the column equilibrated with 0.1% TFA and eluted with 0-80% linear gradient of acetonitrile in 0.1% TFA in 160 min at a flow rate of 1 ml/min. The elution was monitored both by 214 nm and 280 nm absorbence. Each peak was collected manually, and, if necessary, further purified by isocratic RPLC using the same column after being dried and re-dissolved in 0.2 ml of 6 M guanidine HCl-0.1% TFA. The optimum concentration of acetonitrile for separating the peptides each fraction was estimated from the elution pattern of the first HPLC (see equation of Wong et al., Proc. Nat'l Acad. Sci. USA 82:7711-7715 (1985)).
Amino Acid Analysis. The Acyl-Peptide Hydrolase was dialyzed intensively against 0.1 M acetic acid, lyophilized, and hydrolyzed at 110°C for 24 hr and 48 hr in 6 N HCl containing 0.1% phenol. The amino acid composition was determined using a Beckman 6300 Amino Acid Analyzer (see Hirs, Method Enzymol. 11:197-199 (1967)).
Peptide Sequencing. Peptide sequence analyses were carried out using an Applied Biosystems 470A Protein Sequencer and an Applied Biosystems 120A Pth Analyzer (see Hewick et al., J. Biol. Chem. 256:7900-7997 (1981)).
Construction of Probes. Two oligonucleotide probes, YS20.1 and YS17.2, were constructed from the amino acid sequences of two peptides. See Figure 2. The probes were synthesized with an Applied Biosystems 380A DNA synthesizer by using the silica-based solid-phase method (Matteucci et al., J. Am. Chem. Soc. 103:3185-3191 (1981)) and the proton-activated nucleoside phosphoramidite method (Beaucage et al., Tetrahedron Lett. 22:1859-1862 (1981)). The purified oligonucleotides were isolated from the crude synthetic mixtures by PAGE and were labeled with [γ-³²P] ATP to a specific activity of 9 x 10³. YS17.2 and YS20.1 represent pools of 128-fold degenerative oligonuncleotides. The YS17.2 and YS20.1 pools were 17 and 20 nucleotides in length, respectively. The two probes overlap by 12 nucleotides, such that sequential use of the probes to screen a DNA library would effectively screen for a 25 nucleotide piece of APH encoded DNA.

### Example 3--Creation and Screening of the cDNA Library and Sequencing APH Encoded cDNA.

This procedure is illustrated in the right pathway and lower portion of Figure 3.

Rat liver was quick-frozen in liquid N₂ and thawed in quanidine isothiocyanate, and the RNA was purified by centrifugation through CsCl (Chirgwin et al., Biochem 18:5294-5299 (1979)). Poly (A)⁺RNA was selected on oligo (dT)-cellulose (Aviv et al., Proc. Nat'l Acad. Sci. 69:1408-1412 (1972), and was shown to be intact by blotting and hybridization to an actin cDNA probe, pACT1 (Spiegelman et al., J. Biol. Chem. 258:10083-10089 (1983)).

The cDNA was synthesized from 10 µg of poly(A)⁺ RNA by the method of Okayama et al., Mol. Cell. Biol. 2:161-170 (1982), as modified by Gubler et al., Gene 25:263-269 (1983). The cDNA was ligated to EcoRI linkers after the ends of the cDNA were made blunt with T4 DNA polymerase and the internal EcoRI sites were methylated. The cDNA was then digested with excess EcoRI and chromatographed on Sepharose CL-4B. cDNA of greater than 500 bp was pooled and further fractionated on a 0.8% agarose gel in Tris borate buffer. The cDNA migrating at greater than 1 kb was collected with NA-45 paper (Schleicher & Schuell). Ten µg of dephosphorylated gt11 (see Young et al., Proc. Nat'l Acad. Sci. USA 80:1194-1198 (1983)) DNA was ligated to 100 µg of the size selected cDNA in 20 µl and packaged in vitro (see Miniatis et al., Molecular Cloning: A Laboratory Manual (Cold Spring Harbor Laboratories, Cold Spring Harbor, NY) (1982)). E. coli strain Y1088 was infected with recombinant phage, and the library was plated for screening.

Initially, 450,000 clones were plated and screened using YS20.1. Of the 450,000, 27 duplicating positive plaques were observed. Twelve of these 27 were isolated and purified, and then rescreened using YS17.2. Of the 12, only 1 plaque was observed to be a true positive. This clone was designated APH5.2. To confirm that the cDNA insert in APH5.2 was actually encoded for APH, the region of the oligo hybridization site was sequenced by the dideoxynucleotide chain termination method (see Sanger et al., J. Mol. Biol. 94:441-448 (1975)). Translation of the sequence produced the identical peptide sequence from which the oligonucleotide probes were designed.

The cDNA insert in clone APH5.2 was found to be -1.3 kb by restriction enzyme analysis. To get an estimate of the length of the APH gene, the "chromosome walking" method was used to obtain a larger probe, which was then used to hybridize an mRNA coding for APH on a Northern blot. Based on a partial restriction map of APH5.2, a 660 bp Kpn-XmnI fragment was selected. Radiolabeled probes were created by nick-translation and used to screen rat tissue mRNA, prepared according to the Northern blot procedure (Alwine et al., (1979)). The 660 bp probe hybridized to a single mRNA -2.9 kb in size.

Because the 1.3 kb fragment of clone APH5.2 was considerably shorter than the 2.9 kb mRNA, the 660 bp fragment was used to rescreen the rat liver cDNA library. Of the 1.1 million clones plated, 15 duplicating positive plaques were observed. Thirteen of those clones were isolated, and the phage DNA was isolated. The longest chain contained a 2.5 kb cDNA insert and was designated APH36.1.

The phage DNA of APH36.1 was purified and sequenced. The sequencing strategy included the subcloning of endonuclease fragments of APH36.1 cDNA insert into M13, deletion mutants generated by Exonuclease III, or oligonucleotide sequencing primers hybridized to specific sequence regions of the cDNA.

The translated sequence of the 5′ end of APH36.1 cDNA, i.e., the first 40 amino acids, were found to be identical to the amino acid sequence of rat serum albumin. Therefore, APH36.1 was a scrambled clone containing only 2.5 kb of APH coded cDNA.

Figure 1 illustrates the APH36.1 cDNA translated sequence without the rat serum albumin sequence. The singly underscored and cross-hatched sequences are those sequences obtained by sequencing the tryptic and cyanogen bromide fragments of APH. The active site is designated by the cross-hatched area, and each asterisk indicates a possible site of glycosidic modification.

Since each APH subunit has a molecular weight of 80,000, or approximately 700-755 amino acid residues, and since the APH encoded sequence of clone APH36.1 accounts for 2184 base pairs (i.e., 728 amino acids), additional "chromosome walking" was indicated to find the 5′ end of the gene encoded for APH. To that end, a commercially available lambda-gt11 rat liver cDNA library from Clonetech (HRL1001) containing 6.8 x 10⁵ independent clones and an average insert size of 1.1 Kb, was screened with radiolabeled probes created from the Ban 2-Pst I fragment from the extreme 5′ APH coding sequence of clone APH36.1. Clones containing the extended 5′ sequence were isolated and the phage DNA purified. Sequencing of the the phage DNA is done by the dideoxy-chain termination method (Sanger et al., 1975). The extreme 3′ end of the translated sequence is identified by the location of the stop codon (marked by an asterisk).

The sequence of the missing 5′ end of the coding region for Acyl-Peptide Hydrolase is determined by primer extension analysis of Acyl-Peptide Hydrolase mRNA. A synthetic oligonucleotide complementary to the 5′-most end of the coding region of APH 36.1 is sued to prime the synthesis of a cDNA strand. The template in this reaction is either total cellular poly A⁺ RNA or mRNA enriched for Acyl-Peptide Hydrolase message by prior hybrid selection. The cDNA strand synthesized in this manner is directly sequenced by the chemical method of Maxam and Gilbert (Maxam et al., Proc. Natl. Acad. Sci. USA 74:560-564 (1977)).

Although the foregoing refers to particular preferred embodiments, it will be understood that the present invention is not so limited. It will occur to those ordinarily skilled in the art that various modifications may be made to the disclosed embodiments and that such modifications are intended to be within the scope of the present invention.

## Claims (Claims for the following Contracting State(s): AT, BE, CH, DE, FR, GB, GR, IT, LI, LU, NL, SE)

1. Acyl-Peptide Hydrolase (APH), comprising the amino acid sequence depicted in Figure 1.

2. APH according to claim 1 free of native glycosylation.

3. A recombinant DNA molecule which contains a genetic sequence coding for APH according to claim 1.

4. The recombinant DNA molecule according to claim 3, characterized in that said genetic sequence is in cDNA form.

5. The recombinant DNA molecule according to claim 3 or 4, characterized in that said molecule is a vector, especially a plasmid.

6. A host transformed with the recombinant DNA molecule of any of claims 3 to 5, preferably a bacterium, a yeast or a mammalian cell.

7. A method of producing APH which comprises:
(a) providing the DNA molecule of claim 3;
(b) inserting said DNA molecule into a vector;
(c) transforming a host system with said vector;
(d) expressing said APH DNA sequence of said recombinant DNA molecule in said host; and
(e) recovering the APH produced by said expression.

8. A method of hydrolyzing the N-terminal acyl amino acid of an acylated polypeptide, characterized by contacting said polypeptide with the APH of claim 1 or 2.

9. A method of catalyzing the reaction between a derivitized N ^{α}-acetyl amino acid donor and an acceptor with a free α-NH₂, characterized by contacting said donor with said acceptor in the presence of the APH of claim 1 or 2, in a near anhydrous medium.

10. The enzyme according to claim 1 or 2, or an enzmye obtained by the method of claim 7, in immobilized form.

## Claims (Claims for the following Contracting State(s): ES)

1. A method of producing Acyl-Peptide Hydrolase (APH) characterized by
(a) providing a recombinant DNA molecule coding for the amino acid sequence depicted in Figure 1;
(b) inserting said DNA molecule into a vector;
(c) transforming a host system with said vector;
(d) expressing said APH DNA sequence of said recombinant DNA molecule in said host; and
(e) recovering the APH produced by said expression.

2. A method according to claim 1, characterized in that the APH is mammalian, especially rat APH.

3. A method according to claim 1 or 2, characterized in that said genetic sequence is in cDNA form.

4. A method according to one or more of the preceding claims, characterized in that said genetic sequence is a vector, especially a plasmid.

5. A method according to one or more of the preceding claims, characterized in that said host is a bacterium, a yeast or a mammalian cell.

6. A method of hydrolyzing the N-terminal acyl amino acid of an acylated polypeptide, characterized by contacting said polypeptide with the APH obtained according to one or more of the preceding claims.

7. A method of catalyzing the reaction between a derivitized N ^{α}-acetyl amino acid donor and an acceptor with a free α-NH₂, characterized by contacting said donor with said acceptor in the presence of the APH obtained according to one or more of claims 1 to 5 in a near anhydrous medium.

8. A method according to claim 7, characterized in that the enzyme is applied in immobilized form.

## Patentansprüche (Patentansprüche für folgende(n) Vertragsstaat(en): AT, BE, CH, DE, FR, GB, GR, IT, LI, LU, NL, SE)

1. Acyl-peptidhydrolase (APH), die die in Abbildung 1 dargestellte Aminosäuresequenz umfaßt.

2. APH nach Anspruch 1, frei von nativer Glykosylierung.

3. Rekombinantes DNA-Molekül, das eine für APH gemäß Anspruch 1 codierende genetische Sequenz enthält.

4. Rekombinantes DNA-Molekül nach Anspruch 3, dadurch gekennzeichnet, daß die genannte genetische Sequenz in Form von cDNA vorliegt.

5. Rekombinantes DNA-Molekül nach Anspruch 3 oder 4, dadurch gekennzeichnet, daß das genannte Molekül ein Vektor und insbesondere ein Plasmid ist.

6. Mit dem rekombinanten DNA-Molekül nach einem der Ansprüche 3 bis 5 transformierter Wirt, vorzugsweise ein Bakterium, eine Hefe oder eine Säugerzelle.

7. Verfahren zur Herstellung von APH, folgendes umfassend:
(a) Bereitstellung des DNA-Moleküls nach Anspruch 3;
(b) Insertion des genannten DNA-Moleküls in einen Vektor;
(c) Transformation eines Wirtssystems mit dem genannten Vektor;
(d) Expression der genannten APH-DNA-Sequenz des genannten rekombinanten DNA-Moleküls in dem genannten Wirt; und
(e) Rückgewinnung der APH, die mit der genannten Expression produziert wurde.

8. Verfahren zur Hydrolyse der N-terminalen Acylaminosäure eines acylierten Polypeptids, dadurch gekennzeichnet, daß das genannte Polypeptid mit der APH nach Anspruch 1 oder 2 in Kontakt gebracht wird.

9. Verfahren zur Katalyse der Reaktion zwischen einem derivatisierten N^{α}-Acetylaminosäure-Spender und einem Empfänger mit einem freien α-NH₂, dadurch gekennzeichnet, daß der genannte Spender mit dem genannten Empfänger in Gegenwart der APH nach Anspruch 1 oder 2 in einem fast wasserfreien Medium in Kontakt gebracht wird.

10. Enzym nach Anspruch 1 oder 2 oder ein nach dem Verfahren von Anspruch 7 erhaltenes Enzym in immobilisierter Form.

## Patentansprüche (Patentansprüche für folgende(n) Vertragsstaat(en): ES)

1. Verfahren zur Herstellung von Acyl-peptidhydrolase (APH), gekennzeichnet durch
(a) Bereitstellung eines rekombinanten DNA-Moleküls, das für die in Abbildung 1 dargestellte Aminosäuresequenz codiert;
(b) Insertion des genannten DNA-Moleküls in einen Vektor;
(c) Transformation eines Wirtssystems mit dem genannten Vektor;
(d) Expression der genannten APH-DNA-Sequenz des genannten rekombinanten DNA-Moleküls in dem genannten Wirt; und
(e) Rückgewinnung der durch die genannte Expression produzierten APH.

2. Verfahren nach Anspruch 1, dadurch gekennzeichnet, daß es sich bei der APH um Säuger-APH und insbesondere Ratten-APH handelt.

3. Verfahren nach Anspruch 1 oder 2, dadurch gekennzeichnet, daß die genannte genetische Sequenz in Form von cDNA vorliegt.

4. Verfahren nach einem oder mehreren der vorstehenden Ansprüche, dadurch gekennzeichnet, daß die genannte genetische Sequenz ein Vektor und insbesondere ein Plasmid ist.

5. Verfahren nach einem oder mehreren der vorstehenden Ansprüche, dadurch gekennzeichnet, daß der genannte Wirt ein Bakterium, eine Hefe oder eine Säugerzelle ist.

6. Verfahren zur Hydrolyse der N-terminalen Acylaminosäure eines acylierten Polypeptids, dadurch gekennzeichnet, daß das genannte Polypeptid mit der nach einem oder mehreren der vorstehenden Ansprüche erhaltenen APH in Kontakt gebracht wird.

7. Verfahren zur Katalyse der Reaktion zwischen einem derivatisierten N^{α}-acetylaminosäure-Spender und einem Empfänger mit einem freien α-NH₂, dadurch gekennzeichnet, daß der genannte Spender in Gegenwart der nach einem oder mehreren der Ansprüche 1 bis 5 erhaltenen APH in einem fast wasserfreien Medium mit dem genannten Empfänger in Kontakt gebracht wird.

8. Verfahren nach Anspruch 7, dadurch gekennzeichnet, daß das Enzym in immobilisierter Form angewandt wird.

## Revendications (Revendications pour l'(les) Etat(s) contractant(s) suivant(s): AT, BE, CH, DE, FR, GB, GR, IT, LI, LU, NL, SE)

1. Acyl-peptide hydrolase (APH), comprenant la séquence d'aminoacides représentée sur la figure 1.

2. APH selon la revendication 1, exempte de glycosylation native.

3. Molécule d'ADN recombinant, contenant une séquence génétique codant pour l'APH selon la revendication 1.

4. Molécule d'ADN recombinant selon la revendication 3, caractérisée en ce que ladite séquence génétique est sous forme d'ADNc.

5. Molécule d'ADN recombinant selon la revendication 3 ou 4, caractérisée en ce que ladite molécule est un vecteur, en particulier un plasmide.

6. Hôte transformé par la molécule d'ADN recombinant de l'une quelconque des revendications 3 à 5, de préférence une bactérie, une levure ou une cellule mammalienne.

7. Procédé de production d'APH, comprenant:
(a) la production de la molécule d'ADN de la revendication 3;
(b) l'insertion de ladite molécule d'ADN dans un vecteur;
(c) la transformation d'un système hôte par ledit vecteur;
(d) l'expression de ladite séquence d'ADN d'APH de ladite molécule d'ADN recombinant dans ledit hôte; et
(e) la récupération de l'APH produite par ladite expression.

8. Procédé d'hydrolyse de l'acylaminoacide N-terminal d'un polypeptide acylé, caractérisé par la mise en contact dudit polypeptide avec l'APH de la revendication 1 ou 2.

9. Procédé de catalyse de la réaction entre un donneur de N^{α}-acétylaminoacide transformé en dérivé et un accepteur comportant un groupe α-NH₂ libre, caractérisé par la mise en contact dudit donneur avec ledit accepteur en présence de l'APH de la revendication 1 ou 2, dans un milieu presque anhydre.

10. Enzyme selon la revendication 1 ou 2, ou enzyme obtenue par le procédé de la revendication 7, sous forme immobilisée.

## Revendications (Revendications pour l'(les) Etat(s) contractant(s) suivant(s): ES)

1. Procédé de production d'acyl-peptide hydrolase (APH), caractérisé par
(a) la production d'une molécule d'ADN recombinant codant pour la séquence d'aminoacides représentée sur la figure 1;
(b) l'insertion de ladite molécule d'ADN dans un vecteur;
(c) la transformation d'un système hôte par ledit vecteur;
(d) l'expression de ladite séquence d'ADN d'APH de ladite molécule d'ADN recombinant dans ledit hôte; et
(e) la récupération de l'APH produite par ladite expression.

2. Procédé selon la revendication 1, caractérisé en ce que l'APH est mammalienne, en particulier est de l'APH de rat.

3. Procédé selon la revendication 1 ou 2, caractérisé en ce que ladite séquence génétique est sous la forme d'ADNc.

4. Procédé selon une ou plusieurs des revendications précédentes, caractérisée en ce que ladite séquuence génétique est un vecteur, en particulier un plasmide.

5. Procédé selon une ou plusieurs des revendications précédentes, caractérisé en ce que ledit hôte est une bactérie, une levure ou une cellule mammalienne.

6. Procédé d'hydrolyse de l'acylaminoacide N-terminal d'un polypeptide acylé, caractérisé par la mise en contact dudit polypeptide avec l'APH obtenu selon une ou plusieurs des revendications précédentes.

7. Procédé de catalyse de la réaction entre un donneur de N^{α}-acétylaminoacide transformé en dérivé et un accepteur comportant un groupe α-NH₂ libre, caractérisé par la mise en contact dudit donneur avec ledit accepteur, en présence de l'APH obtenue selon une ou plusieurs des revendications 1 à 5, dans un milieu presque anhydre.

8. Procédé selon la revendication 7, caractérisé en ce que l'enzyme est utilisée sous forme immobilisée.
